# EUROPEAN PATENT SPECIFICATION

(11) **EP 2 978 377 B1**
(45) Date of publication and mention of the grant of the patent: **05.05.2021**
(21) Application number: 13724157.6
(22) Date of filing: 26.03.2013
(51) Int. Cl.: G01N 23/041

(54) **METHOD OF PHASE GRADIENT RADIOGRAPHY AND ARRANGEMENT OF AN IMAGING SYSTEM FOR APPLICATION OF THE METHOD**
VERFAHREN ZUR PHASENGRADIENTENRADIOGRAFIE UND ANORDNUNG EINES ABBILDUNGSSYSTEMS ZUR ANWENDUNG DES VERFAHRENS
MÉTHODE DE RADIOGRAPHIE À GRADIENT DE PHASE ET AGENCEMENT DE SYSTÈME D'IMAGERIE POUR L'APPLICATION DE LA MÉTHODE

(43) Date of publication of application: 03.02.2016
(73) Proprietor: Institute of Experimental and Applied Physics, 110 00 Praha 1 (CZ)
(72) Inventor: JAKUBEK, Jan, 267 05 Hyskov (CZ); KREJCI, Frantisek, 679 62 Kretin (CZ)
(74) Representative: Kendereski, Dusan
(86) International application number: PCT/CZ2013/000045
(87) International publication number: WO 2014/154188

(56) References cited:
- GB-A- 2 441 578
- US-A1- 2012 041 679
- KREJCI FRANTISEK ET AL: "Hard x-ray phase contrast imaging using single absorption grating and hybrid semiconductor pixel detector", REVIEW OF SCIENTIFIC INSTRUMENTS, AIP, MELVILLE, NY, US, vol. 81, no. 11, 5 November 2010 (2010-11-05), pages 113702-113702, XP012145818, ISSN: 0034-6748, DOI: 10.1063/1.3499372
- KREJCI F ET AL: "Single grating method for low dose 1-D and 2-D phase contrast X-ray imaging", JOURNAL OF INSTRUMENTATION, INSTITUTE OF PHYSICS PUBLISHING, BRISTOL, GB, vol. 6, no. 1, 11 January 2011 (2011-01-11), page C01073, XP020203529, ISSN: 1748-0221, DOI: 10.1088/1748-0221/6/01/C01073
- KROUPA M ET AL: "Optimization of the spectroscopic response of the Timepix detector", JOURNAL OF INSTRUMENTATION, INSTITUTE OF PHYSICS PUBLISHING, BRISTOL, GB, vol. 7, no. 2, 27 February 2012 (2012-02-27), page C02058, XP020218912, ISSN: 1748-0221, DOI: 10.1088/1748-0221/7/02/C02058
- IGNATYEV K ET AL: "Effects of signal diffusion on x-ray phase contrast images", REVIEW OF SCIENTIFIC INSTRUMENTS, AIP, MELVILLE, NY, US, vol. 82, no. 7, 8 July 2011 (2011-07-08), pages 73702-73702, XP012146565, ISSN: 0034-6748, DOI: 10.1063/1.3606442

## Description

### Field of Invention

The invention deals with a radiation imaging method including steps of generation of at least one radiation microbeam by a radiation source, radiation penetration through the examined object and its caption by a detector consisting of a system of pixels, while the difference between the position of a non-refracted microbeam detected without the examined object and the position of a refracted microbeam detected with the examined object defines refraction angle in a particular point of the examined object expressing the size of local gradient of refractive index corresponding to the phase gradient image (PGI) of the examined object.

### State of the Prior Art

A technology for characterization of inner structures of objects is already available nowadays. These technologies include non-destructive imaging systems based on phase contrast. Phase sensitive radiography with X-ray radiation (or neutrons or other applicable radiation) is an efficient non-destructive imaging technique enabling obtaining contrast images of samples even in situations when standard absorption radiography does not provide sufficient quality (e.g. imaging soft tissue or composite material structures). The method is generally based on measurement of phase shift of radiation penetrating the sample. The phase shift occurs inside the sample thanks to uneven distribution of refraction index in the sample. The phase shift then causes change of radiation propagation direction - this phenomenon is known as refraction in the visible light spectrum. The refraction angle size depends on the extent of refraction index change at a particular point of the sample. The measurement of this angle at multiple points of the sample enables creation of an image of distribution of refraction index changes in the sample and thus visualization of its inner structure.

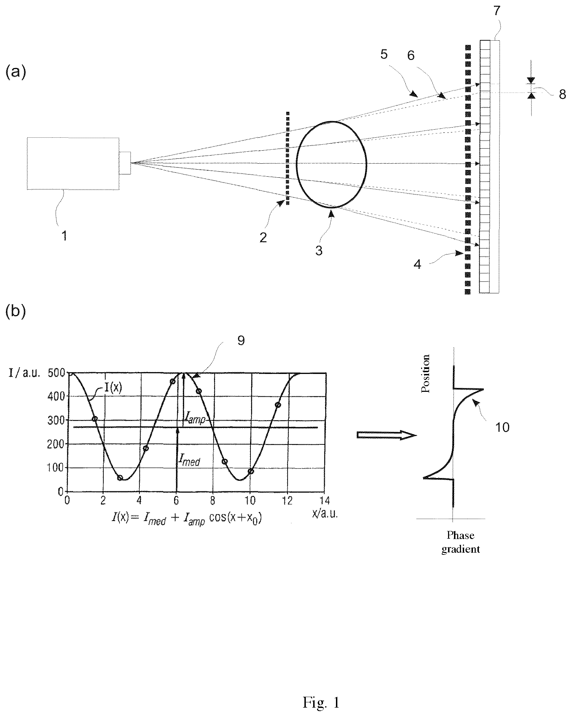

The existing systems as well as the present invention will be described with X-ray radiation, implementations with different radiation types, e.g. neutrons are analogical. Fig. 1 shows one of the existing systems using a solution with two gratings (a grating interferometer). This system includes a spatially coherent radiation source (e.g. micro-focus X-ray tube), a grating forming microbeams of the applied radiation, examined object, detector grating, refracted microbeam of rays (with an object), non-refracted microbeam of rays (without an object), pixel detector, while the difference in the positions of the refracted and non-refracted microbeams is illustrated by pos. 8. Characteristic intensity oscillation serving for phase shift evaluation observed in each pixel when one of the gratings is being scanned is illustrated by pos. 9 and the phase gradient image (PGI) obtained from measured data is in pos. 10.

Phase sensitive radiography systems are usually based on refraction angle measurement by means of a system of gratings inserted between the radiation source and the detector, see e.g. invention applications [1] [2] and references [3], [4], [5].

These systems require radiation sources with high degree of spatial coherency, which means that each point of the examined object is irradiated with a very narrow angular spectrum of the applied radiation. This source might be e.g. a micro-focus X-ray generator, or a combination of a standard-sized non-coherent X-ray source and suitable collimation performed right behind the radiation source. Systems based on synchrotron radiation usually achieve sufficient spatial coherency by a long distance between the radiation source and the examined object.

Spatially coherent radiation passes through the grating, which forms it into a set of microbeams directioned to irradiate a 2D pixel detector. The microbeam formation by the grating can be realized by absorption, i.e. the grating acts as a matrix of micro collimators, or by a phase grating utilizing the so-called Talbot effect, see reference [6]. In this case, the grating modulates the phase of incident radiation in the space so that the required intensity modulation is observed at a certain distance behind the grating thanks to the interference of rays with different phase shift.

If an examined object with non-homogenous refraction index distribution is located between the microbeam source and the detector, the direction of the microbeams penetrating through the examined object changes according to local refraction index gradient in the examined object. The position where the microbeams fall on the detector also changes as a consequence of the change of the microbeams direction. The difference of the position of the non-refracted microbeam (without an object) and the refracted microbeam (with an object) corresponds with the refraction angle in the particular point and expresses the size of local refraction index gradient in the examined object. If there are a lot of such points, a map of phase gradient of the examined object, i.e. its phase gradient image (PGI) can be assembled. The ability to measure exactly the change of the position between refracted and non-refracted microbeams is thus the most important factor defining quality of the described imaging system.

The size of the projected microbeams in the systems using a grating interferometer is typically 2 µm, as shown in Fig. 1 and e.g. invention applications [1] [2] and references [3] and [4], so spatial resolution of the existing detectors is insufficient for a direct determination of the change of position of refracted microbeams. This is why such systems are equipped with an additional (analyzing or detector) absorption grating located directly in front of the detector. For the retrieval of the position change of the projected microbeams, several radiographs with various gratings positions have to be measured, see references [3] and [4]. In this process, one of the gratings is shifted equidistantly within one own period, which enables observation of characteristic intensity oscillation in each pixel as a function of the position of the grating being moved. The resulting microbeam shift and thus also the phase gradient image is then obtained by assessment of parameters of the oscillation curve in each pixel (the phase gradient is given by the oscillation curve shift in the cases with and without the examined object, i.e. parameter xo - see Fig. 1b).

Very low time resolution, when the examined object has moreover to be kept absolutely immovable during all the acquisitions, is a disadvantage of this approach. Another crucial limitation of the system is given by the fact that for the measurement of each individual radiogram detection of a very high number of radiation particles (thousands and more) sent to each microbeam is necessary. The total number of used radiation particles is moreover increased by the necessity to perform several exposures during the grating scanning procedure. The number of particles used for obtaining the final phase image actually determines the total radiation dose absorbed by the object. If live tissue is the sample, the adverse biological effects of the absorbed dose limit applicability of the described imaging method. This factor has key importance in X-ray radiation and planned applications, e.g. in mammography.

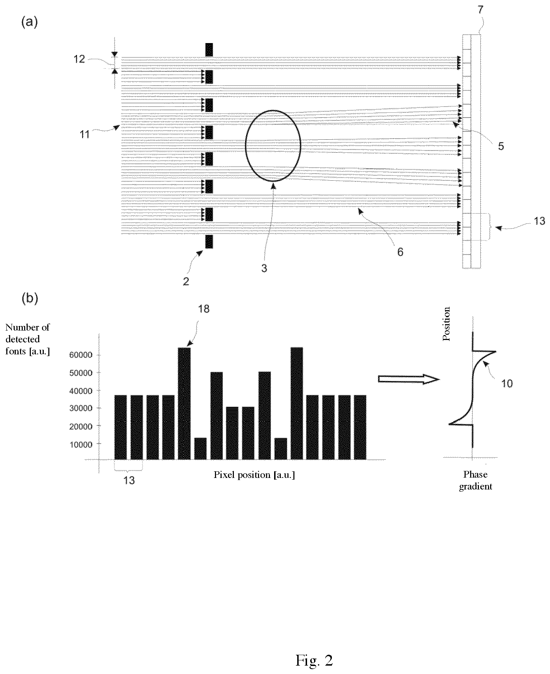

The necessity to perform more exposures to retrieve the phase information is eliminated in the system described in reference [5], see Fig. 2. The system is based on the combination of a spatially coherent radiation source, one absorption grating and a 2D pixel detector which has practically ideal pixel response and enables single-photon detection. The individual system components are in this case arranged to form the incoming radiation from the spatially coherent source, identified by pos. 11 in Fig. 2, by the absorption grating so that the created beams irradiate systematically two adjacent pixels. Without the examined object, a half of the beam intensity is recorded by one pixel and the other by another pixel. After insertion of an examined object, refraction of beams occurs and their position on the detector is changed. This change appears as a change of the proportion of intensities in appropriate adjacent pixels forming a detection multiplet, in which phase information is evaluated, see reference [5]. In this case the phase gradient image is identified in Fig. 2b as position 10 and the record of intensity (numbers of photons) of the incident radiation in the individual pixels used for the PGI calculation by position 18.

A substantial factor of this system is that only the detected photons counted just in one pixel of the respective multiplet are important for the beam position assessment. These photons actually change the proportion of intensities in the appropriate pair of pixels, from which the position of the incident beam is directly calculated. The photons that cause the detected event in both the pixels thanks to signal sharing (particularly those falling exactly on the boundary of pixels) bear no phase information and only increase the background in the usable signal. Consequently, the width of the beams formed by the absorption grating (pos. 12 in Fig. 2a), given by the size of openings in the grating and by geometric enlargement, is selected by about one order higher than in the above described grating interferometer approach. As a result, to be able to retrieve the phase gradient image (PGI). A full intensity radiogram requiring typically 10⁴ and more detected events per pixel has to be measured.

The effect of signal (and particularly charge) sharing is generally considered adverse in radiation imaging and development in the field of digital detectors and whole imaging systems is driven by endeavours to eliminate the effect to the maximal extent (see e.g. patent application [7] and reference [8]). The effect of signal sharing also reduces the quality of detected phase images in the above described current phase radiography systems.

### Summary of the Invention

The aim of the invention is to propose a method of phase gradient radiography according to independent claim 1 and an arrangement of a radiographic imaging system according to independent claim 5 that enables acquisition of phase images with substantially reduced radiation intensity. In a limit case, detection of just one radiation particle per each image element (pixel) is sufficient.

The above described drawbacks are eliminated by a radiation imaging method including steps of generating at least one radiation microbeam by a radiation source, radiation penetration through an examined object and its caption by a detector. The difference between the position of a non-refracted microbeam detected without the examined object and the position of a refracted microbeam detected with the examined object defines a refraction angle in a particular point of the examined object, expressing the size of a local gradient of the refractive index corresponding to phase gradient image (PGI) of the examined object. The principle of invention is based on the fact that the radiation on the way from the radiation source to the detector passes through a directing element which transmits a radiation microbeam with crosswise dimension and directs the radiation microbeam to the boundaries between pixels of the detector. The incident radiation particle generates an analogue signal in the pixels adjacent to the boundary and each of the pixels detects the appropriate share of the signal. The share of the analogue signal is the effect of the same photo event causing a detected signal in a plurality of pixels adjacent to a boundary. The non-refracted microbeam generates approximately the same share of analogue signal in each pixel of the multiplet, and then after object insertion the microbeam is refracted in the particular point of the object and the analogue signal is unequally distributed and shared among pixels adjacent to the boundary within one multiplet. The difference of the position between the refracted microbeam and the non-refracted microbeam is directly derived from the change of the analogue signal share within the appropriate pixels adjacent to the boundary (the so-called multiplet). The phase gradient in the particular object point is thus directly derived from the analogue signal. This method enables the evaluation of the refraction angle and the corresponding phase gradient separately for each detected particle, which significantly reduces the number of detected particles necessary for PGI formation. In the limit case, only one particle has to be detected in each multiplet to obtain a PGI of the examined object, i.e. the retrieval of the phase gradient information can be performed for each individual detected particle in the refracted radiation microbeam using a signal generated by each individual detected particle (with the advantage the size of the deposited charge).

The imaging system can be combined with computed tomography (CT) methods to provide 3D imaging of inner structure of examined samples. To detect the space distribution of the refraction index in the examined object by means of computed tomography methods, a set of projections is measured, namely phase gradient images (PGIs), obtained by the above method for various rotation angles of the examined object. Projections under various angles are ensured by the rotation of the examined object placed on a rotating table, where the other elements, the source, the directing element and the detector are fixed. Projection under various angles can also be ensured by means of the radiation source, the directing element and the detector arranged on a rotating gantry orbiting the examined object on a fixed table.

The arrangement of the radiographic imaging system for the application of the method, consisting of the spatially coherent radiation source, space for the examined object and the detector consisting of the set of pixels is based on the fact that it has the directing element that directs the microbeams to the area of boundary between at least two detector pixels to form a multiplet. The crosswise dimension of each microbeam of the radiation falling on the detector is c times smaller than the size of the detector pixel, where c is 2 - 1000 and c > 20 is advantageous. The detector has the ability in each pixel of the multiplet to record an appropriate part of the analogue signal arisen after the detection of each individual radiation particle. The detector is arranged to detect signal share defined as the effect of the same photo event causing a detected signal in a plurality of pixels adjacent to a boundary.

The radiographic imaging system thus consists of a pixel detector of radiation, which is able to detect the analogue signal arisen after the detection of each individual radiation particle, while the size of the detector pixels is between 0.1 and 500 µm. If a particle of the applied radiation (a particle in the non-refracted microbeam is indicated as pos. 23 and in the refracted microbeam as pos. 27) impacts close to the boundary between several pixels forming the multiplet (doublet, triplet, quadruplet etc.), each of these pixels detects the appropriate part of the signal. This effect is called sharing within the multiplet.

The directing element directing microbeams to the area of boundary between at least two detector pixels forming the multiplet is usually realized by a grating or micro-capillary X-ray optics.

The system also includes a system of microbeams of the applied radiation: a refracted microbeam (its direction is affected by the examined object), a non-refracted beam (its direction is not affected by the examined object). Thanks to mutual position of the coherent radiation source (e.g. micro-focus type X-ray source), a grating forming the microbeams and the detector, each microbeam always irradiates a common boundary of (at least two) adjacent detector pixels while sharing of signals within such multiplets occurs. The difference between the refracted and the non-refracted microbeams can be determined with high accuracy from the change of signal sharing within the appropriate detector multiplet. Registration of at least one radiation particle in the multiplet is sufficient for that. For evaluation of the difference between the refracted and the non-refracted microbeams, calculation of the centre of mass of the signal detected within the multiplet for example can be used.

Location of the examined object in front of the detector causes a change of the direction of propagation of the radiation microbeams and a shift of their tracks within the detector multiplets. The size of such a shift is evaluated for each multiplex separately and the arrangement of the detected shifts into a map corresponding with the original arrangement of the microbeams results in a phase gradient image (PGI) of the examined object.

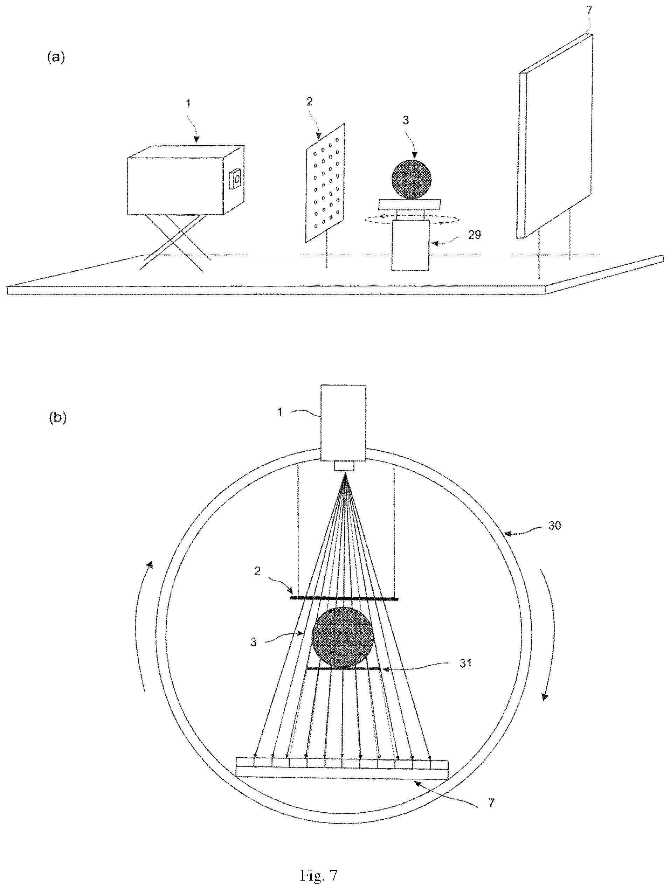

If a set of PGIs is measured by this procedure for various angles of rotation of the examined object (projections), 3D distribution of refraction index in the examined object can be evaluated by known methods of computed tomography. Projections under different angles are ensured by known methods, i.e. either by rotating the examined object (the microbeams and the detector are fixed in this version, see Fig. 7a) or by the system where a set (gantry) of the remaining system elements, i.e. the radiation source, the grating and the detector rotate around the fixed examined subject, see Fig. 7b.

If the energy of the applied X-ray radiation is controlled by elements included in the source or detected by detector elements enabling determination of energy of each detected particle, the method and the arrangement according to this invention can be used for energy sensitive phase gradient imaging enabling identification of materials used in the structure of the examined object.

A significant advantage of the solution according to this invention is that the method and arrangement of the radiographic imaging system reduces radiation dose absorbed by the sample up to several orders and considerably shortens the measurement time.

Another advantage of the method and arrangement of the radiographic imaging system according to this invention is higher sensitiveness to the measured phase information and the possibility to design a system with substantially shorter distance between the imaged object and the detector, which leads to considerable advantages that will be described below.

Compared to the existing phase radiography systems using grating interferometer, as seen in Fig. 1, the present system has the following basic differences and subsequent advantages:
- the present radiographic imaging system contains no analysing (detector) grating. The phase gradient image is obtained from just a single exposure, which means that it is not necessary to shift one of the gratings and take several exposures to evaluate PGI of the examined object. This considerably improves the time resolution of the method. The ability to acquire an image of an examined object with one setting of the radiographic imaging system is crucial for imaging unstable biological objects either in planned clinical application (e.g. mammography) or imaging of unstable samples in biology.
- elimination of multiple exposures reduces the radiation dose given to the examined object. Except for reduction of the number of exposures, the deposited dose is considerably reduced also by further aspects of the proposed radiography imaging system, which are discussed in comparison with the system described in reference [5].
- the fact that an analysing (detector) grating has significant absorption also in places where the grating is considered to be transparent contributes to dose increase for the investigated sample. To achieve sufficient number of photons reaching the detector, in systems operating with grating interferometer the intensity or exposure time has to be adequately increased.
- elimination of the detector grating highly simplifies the whole radiography system. Manufacturing of a detector grating itself is technologically demanding. Its manufacturing for higher X-ray radiation energy (over 50 keV) and for larger areas remains extremely complicated, if possible at all. Both the above properties are still crucial for intended medical applications.
- the present radiographic imaging system with one grating can be directly used for evaluation of phase information in two directions from a single exposure, which can be effectively used for improving detected image quality (it is for example a way to further reduction of dose, as already demonstrated).

Compared to the system described in reference [5], (system using an absorbing grating and hybrid semiconductor pixel detector operated in the counting mode), the present radiographic imaging system has the following basic differences and subsequent advantages:
- the present radiographic imaging system compared to the system described in reference [5] enables PGI acquisition with significantly reduced radiation dose. In a limit case, detection of just one radiation particle per each image element (pixel) is sufficient. In this way, the radiographic imaging system according to the invention reduces radiation dose absorbed by the examined object by up to several orders of magnitude and remarkably reduces measurement time.
- compared to the system described in reference [5] the radiographic imaging system uses a different grating. Its parameters and location in the system are arranged so as the incidence area of the microbeams on the detector is at least 100 - 1000 times smaller than in the system described in reference [5]. A different method of PGI acquisition is the reason. A relatively wide beam is projected in the system described in reference [5] - the beam width at the image measurement plane is comparable to the size of a pixel of the used semiconductor pixel detector 55 µm. In the system described in reference [5] without a sample a half of the intensity is measured by one pixel and the other one with another one. Refraction causes a change of proportion of the intensities in the appropriate pixels, from which the required position change of the projected beam can be obtained. It is thus necessary to measure in principle a full intensity radiograph for PGI evaluation, while each pixel typically registers 10⁴ and more detected photons.

- pixel multiplets are also used in the newly designed radiographic imaging system, but the signal serving for PGI calculation is not the intensity of the incident photons. It is the signal generated by each individual detected particle (e.g. the amount of the deposited charge). If a detector is able in each pixel of the multiplet to record the value of shared analogue signal generated by each individual detected particle (such a detector utilizing the charge sharing effect is described in the text), then each such a detected particle bears the required phase gradient information directly in itself.
- detection of a single particle in each multiplet is principally sufficient for PGI measurement in the present radiographic imaging system. Experience has shown that for formation of images of sufficient quality this number has to be increased because of statistical character of photon interaction in the object and the detector. Nevertheless, the number of phonons necessary for formation of a PGI of the same quality is still at least 100 - 1000 times lower compared to the system described in reference [5].
- the newly designed radiographic imaging system uses photons that generate shared signal during their incidence on pixels boundary regions. The design of the new system thus maximizes the number of shared events. On the other hand, the system described in reference [5] requires maximizing of single-pixel events and suppression of shared events. Actually, only the photons that formed above-threshold signal in only one pixel of the used multiplet contribute to PGI formation in the system described in reference [5]. Even in the system described in reference [5] signal sharing occurs parasitically during pixel boundary irradiation. All the impacted pixels with signal above a certain threshold value add one to the measured intensity in such a situation. From the point of view of PGI formation this is just an increase of the background level in the observed signal.
- the proportion of single-pixel and shared events generated by the used X-ray radiation determines favourableness of both the approaches. Creation of shared events can be together with the photon place of interaction in relation to the pixel area also influenced by numerous factors such as sensor thickness, X-ray radiation energy, detector pixel size etc. Practical phase radiation imaging applications plan use of harder X-ray radiation requiring more sophisticated (thicker) sensor. Both the mentioned factors (energy, thickness) lead to higher proportion of shared detected events, which makes the proposed system more advantageous. Finer pixelation of the sensor also leads to higher number of shared events.
- one of the existing limitations of the system described in reference [5] is that requirements for the adjustment of the projected beams in relation to the pixel matrix are very stringent. The distribution of the projected beams actually has to copy the pixel matrix of the detector used. The result is that transition of the approach to detector systems with irregular pixel arrangement (e.g. multichip detectors) is very difficult. Stringent requirements for the adjustment of the projected beams and the detector matrix also complicate transition of the method described in reference [5. to larger field of views. Using the applied geometry with a point-like source, the projected beams with the same phase actually occur on spherical surface which should correspond with the detector in plane geometry. The newly designed approach moderates conditions for the adjustment of the projected microbeams in relation to the matrix of detecting elements, if multipixel multiplets are generated by the radiation (reliably proven for neutron radiation).
- according to the latest measurements the newly designed method shows higher sensitivity to measured phase gradients compared to the system described in reference [5], i.e. can be used for measurement of smaller phase gradients.
- the system described in reference [5] requires the distance source-detector typically 40 - 50 cm. Higher sensitivity to measured phase information in the designed system enables significant reduction of this distance (at least twice according to the latest observation). This leads to higher mechanical stability of the system, which is crucial in imaging requiring longer exposure stability, e.g. in tomography measurement.

### Brief description of the Drawings

The invention is illustrated by the enclosed drawings, where Fig. 1 a shows the existing technical imaging system solution using a grating interferometer, Fig. 1b shows measured phase gradient and an example of oscillation of detected intensity in one detector pixel, which is observed during the scanning with one of the gratings, Fig. 2 shows another variant of technical solution of imaging system with one absorption grating, Fig. 2b shows measured phase gradient and demonstration of intensity record in one detector line used for phase gradient calculation, Fig. 3 shows arrangement of the radiographic imaging system according to the invention, Fig. 4 shows possibilities of microbeam incidence on the detector and creation of appropriate multiplets, Fig. 5a shows the effect of charge sharing in the pixel detector Timepix with a planar semiconductor sensor in the case of even charge sharing and Fig. 5b shows the effect of charge sharing in pixel detector Timepix with planar semiconductor sensor in the case of unequal charge sharing and Fig. 6 shows the effect of charge sharing in the pixel detector Timepix with a sensor with 3D electrodes, Fig. 7 shows schematically the possibilities of measuring phase gradient images (PGIs) for various angles of object rotation (projections), from which the space distribution of refraction index in the examined object can be obtained by computed tomography methods, Fig. 7a shows a variant where the examined object rotates and the other elements (source, grating, detector) are fixed and Fig 7b shows a system embodiment where a system (gantry) containing the system elements, i.e. radiation source, grating and detector rotates around the fixed examined object.

### Exemplary Embodiments of the Invention

The new radiographic imaging system will be described in the following text on the example of X-rays, the use of other radiation types (e.g. neutrons) is analogical.

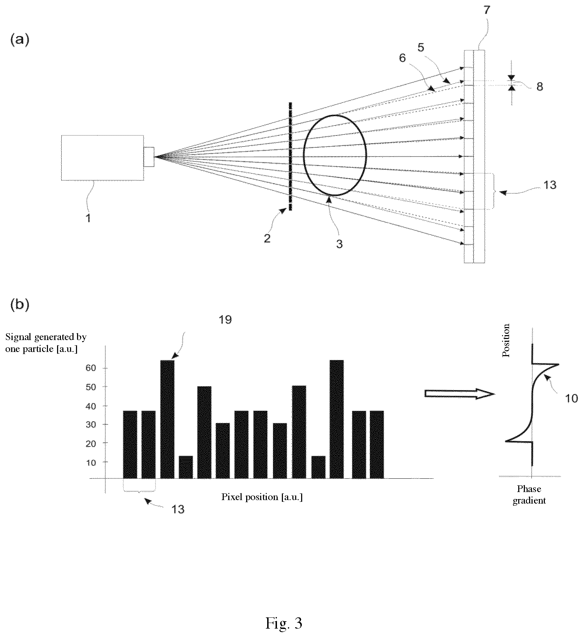

The radiographic imaging system according to this invention is based on the geometry described in Fig. 3. The designed radiographic imaging system consists of a spatially coherent X-ray source **1**, which is a source of X-ray beams, in front of which a directing element in the form of a grating **2** forming microbeams falling on a pixel detector **7** is arranged, while these microbeams are a refracted radiation microbeams **5** (their direction is affected by the examined object **3**) and a non-refracted radiation microbeams **6** (their direction is not affected by the examined object **3**).

An example of spatially coherent source of X-ray radiation is a micro focus type X-ray source **1**, an X-ray generator with a larger incoherent radiation spot in combination with an additional collimator forming a matrix of spatially coherent sources, an X-ray generator with structured anode or e.g. an X-ray generator with a structured beam of excitation electrons. Spatial coherence can also be achieved by placing a large intensity source at a longer distance from the examined object **3** (e.g. at up to 100 metres distance) - this implementation is possible if the proposed system is used at synchronous radiation sources.

The proposed radiographic imaging system arrangement uses one grating **2,** which forms the incident coherent radiation into extremely thin radiation microbeams, which have specific arrangement in relation to the used detector **7** (described below). The crosswise dimension **17** of each microbeam incident on the detector **7** defines indefiniteness in the direction of propagation of penetrating radiation, which is why it is chosen as small as possible (usually 2 µm, or less if the technology of the grating **2** type allows). The grating **2** forming the microbeams can be absorbing, i.e. it works as a system of collimators. Such an absorption grating **2** is usually made by suitable spatial application of strongly attenuating material (e.g. gold) on carrying material, which is practically transparent for the chosen radiation. The absorption grating **2** manufacturing technology usually involves photoresist application, exposure through an optical mask, wet etching and galvanic electroplating. A phase grating using the Talbot effect can also be used as grating **2** to form the microbeams.

Produced microbeams are oriented to a 2D pixel detector **7,** which includes two picture elements (pixels) **14** in such arrangement and size that each incident microbeam can be identified. Determination of the difference of position **8** of the refracted microbeam **5** and the non-refracted microbeam **6** is based on the principle of signal sharing between two or more pixels **14** of the detector **7.** In this process the detected ionising particle **23** generates a signal, which is registered by several adjacent pixels **14.** Such a signal can be for example electric charge **25** in the detectors **7** based on ionisation of detecting material (e.g. a semiconductor) or light in detectors using scintillation materials as the sensor.

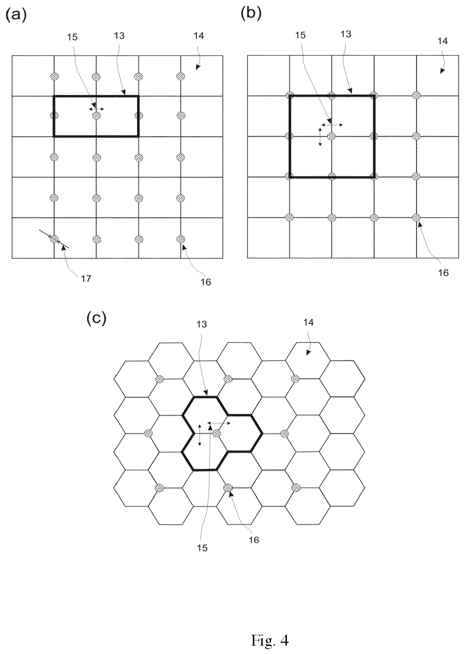

Unlike the existing solutions which try to eliminate the charge sharing effect to the maximum extent, the radiographic imaging system according to the invention is designed so as it only generates especially the shared events and the phase gradient image **10** (PGI) is obtained solely from them. For this purpose the proposed imaging system and its elements - the spatially coherent radiation source **1,** the grating **2** forming the refracted microbeams **5** and the non-refracted microbeams **6** and the detector **7** - are designed and arranged so as the projected refracted microbeams **5** and non-refracted microbeams **6** systematically illuminate in certain positions **16** very limited area (diameter of the beam track **17** on the detector **7** is typically several micrometres) on the boundaries of adjacent pixels **14** of the detector **7.**Some possibilities of arrangement of incident radiation microbeams in relation to the matrix of detector **7** pixels are illustrated in Fig. 4a, Fig. 4b and Fig. 4c. This figure shows three examples; however, a lot of other analogous variants exist.

The incident microbeam generates a signal in a particular group of pixels **14** referred to as multiplets **13.** Those in Fig. 4a, Fig. 4b and Fig. 4c are doublets (a), triplets (c) and quadruplets (b). Pairs of pixels **14** in the first figure (a) enable determination of the shift between the refracted microbeam **5** and the non-refracted microbeam **6** in one direction **15.** Bigger multiplets **13** than doublets enable determination of the shift between the refracted microbeam **5** and the non-refracted microbeam **6** in two independent directions see Fig. 4b and 4c. This means the possibility of simultaneous measurement of two phase gradient images (PGIs) **10** for these directions in phase sensitive imaging.

The radiographic imaging system creates a set of refracted radiation microbeams **5** and non-refracted radiation microbeams **6** which position is evaluated on the detector **7** in pixel multiplets **13** thanks to a purposefully created effect of signal sharing. It is actually possible with correct interpretation of the share signal to determine the position of an incident particle with high subpixel accuracy upon knowledge of the size of the detected signal in several adjacent pixels **14,** as demonstrated in references [9] [10] and [11]. In the proposed arrangement of the radiographic imaging system this property is used for evaluation of the change of direction of the non-refracted microbeam **6** (without the examined object **3**) and the refracted microbeam **5** (with the examined object **3**).

The radiographic imaging system has to be calibrated without the examined object **3** before use. Mutual position of non-refracted microbeams **6** and the detector **7** is tuned in the calibration phase so as each microbeam is directed (absolutely directly in an ideal case) to boundary between two or more pixels **14** of the detector **7.** The signal generated by each microbeam in the detector **7** is thus shared by two or more pixels **14** in the particular multiplet **13.** Division of signals in the individual pixels **14** is recorded and serves as reference for evaluation of a phase gradient map, creating the phase gradient image (PGI) **10** of the examined object **3.**

During measurement the examined object **3** is located between the source **1** of non-refracted radiation microbeams **6** (i.e. after the grating **2**) and the detector **7.** Unless the examined object **3** is subject to radiation damage effects, it may be also located before the grating **2** forming non-refracted microbeams **6.** Refraction index changes in the examined object **3** cause diffraction of non-refracted microbeams **6** (they become refracted radiation microbeams **5**), and thus a shift of their tracks in the detector **7** plane. Even very small shift of each microbeam actually causes a larch change on sharing signals between the pixels **14** of the particular multiplet **13.** Evaluation of signal sharing in the appropriate multiplets **13** enable determination of the shift **8** of the track **16** of each refracted beam **5** in relation to the position of the non-refracted microbeam **6** and thus compilation of a phase gradient map of the particular examined object **3.**

The proposed radiographic imaging system enables quantitative measurement of phase gradient and thus also refraction index distribution in the examined object **3.** If a set of phase gradient images is measured for various angles of rotation of the examined object **3,** 3D distribution of refraction index in object **3** can be evaluated by computed tomography methods. This transition from 2D projection to spatial distribution of refraction index in examined object **3** is the same as in the existing phase radiography systems. Projection under different angles are ensured either by rotating the examined object **3,** while the radiation source **1**, the grating **2** and the detector **7** are fixed, or on the other hand by the implementation of a system where a set (gantry) of the remaining radiographic imaging system elements rotates around a fixed examined object **3.**

To measure the position of a projected microbeam, the detector **7** has to be able to determine the quantity of the signal **19** (e.g. charge or light), that was registered by each imaging element (pixel) **14** during interaction of the detected particle. Optimally (but not necessarily) the detector **7** is able to measure the signal independently for each individual detected X-ray photon.

Unless the electronics of the detector **7** is able to process information on each interacting particle, the microbeam shift in the multiplets **13** is evaluated from an integral signal, which is generated by the interacting particles in appropriate pixels **14** during certain exposure time. A CCD camera equipped with a scintillation sensor is an example of such a pixel detector **7.** Light converted by the CCD camera to electric charge is the shared signal in this case.

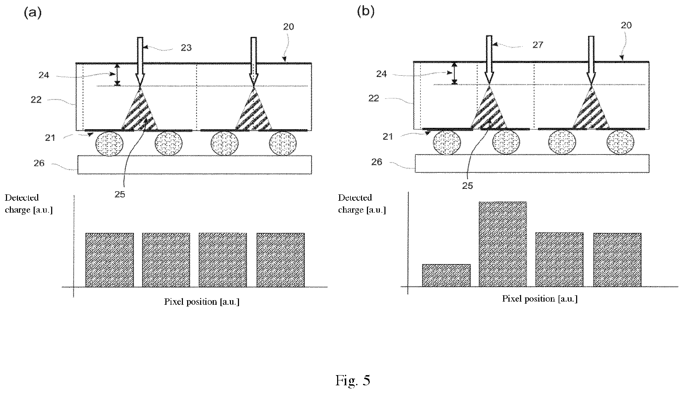

The detector **7,** which can be effectively used in the proposed system and which enables evaluation of the signal generated by each interacting particle is a semiconductor pixel detector **7** "Timepix" see reference [12]. As obvious from Fig. 5a the detector **7** consists of two semiconductor chips. The first, a semiconductor sensor **22,** serves as sensitive volume of the detector **7.** It is usually made of silicon, less frequently of materials such as CdTe and GaAs. The semiconductor sensor **22** is equipped with an upper common electrode **20,** while the other electrode **21** is segmented to form a matrix of contacts evenly covering the area of the sensor **22** of the detector **7.** Each pixel **14** of the segmented electrode **21** is conductively connected to the second (readout) chip **26** "Timepix" on which evaluation electronics is integrated for each pixel **14.**

In the case of the "Timepix" detector **7** operating in a Time-over-Threshold mode these electronics works as independent spectroscopic line capable of evaluation of the quantity of charge **25** deposed in interaction of a single ionising particle to each pixel **14.** The shared signal capable of evaluating the position of incident microbeam is thus electric charge in this case.

Illustration of the charge sharing in the "Timepix" detector **7** with planar semiconductor sensor **22** and the principle of measurement of projected beam shift with sub-pixel accuracy is shown in Fig. 5. Ionising particle **23,** (X-ray photon) creates charge **25** in emptied area of the semiconductor sensor **22,** which is collected by electric field towards segmented electrodes **21.** If this charge **25** occurs near the boundary between two pixels **14,** the appropriate affected adjacent pixels **14** forming a detection multiplet **13** detect its particular part.

The individual system components are aranged so that if refraction does not occur (situation without the examined object **3,** see Fig. 5) the sharing of charge generated by incoming particle **23** is practically equal. After insertion of the examined object **3** which causes slight shift of the projected microbeam position, the signal is no more shared evenly (see Fig. 5b) and the sizes of the signals generated by the refracted particle **27** on the correspoinding adjacent contacts enable determination of position change of interaction of the particle in the detector **7** with accurracy, which is higher than the indefiniteness given by the pixel **14** size.

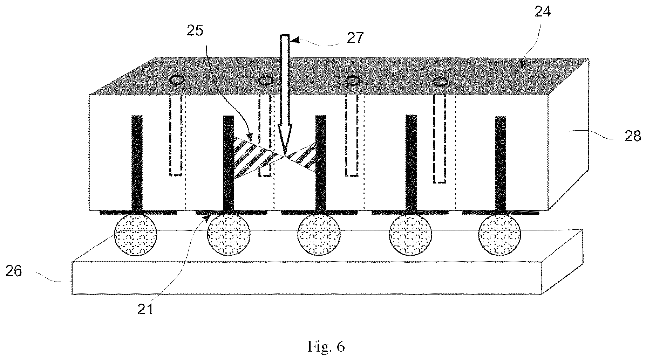

For particles with uneven penetration depth **24** (typical for X-ray radiation), it is advantageous to use the "Timepix" readout chip **26** in combination with a sensor where the segmented electrode **21** is arranged that its pixels **14** fill up the whole sensitive volume of the sensor **22** (sensor with 3D electrodes **28,** see Fig. 6). Specific location of electrodes **21** ensures that the proportion of the collected charge **25** in the adjacent pixels **14** is in fact independent on penetration depth **24.** This detector **7** can be advantageously used in the proposed radiographic system as it improves the accuracy of determination of the position of the incident microbeam **27** by the shared signal **19** weighing method.

For determination of refraction index spatial distribution, a set of phase gradient images (PGI projections) is measured by the described method for various rotation angles of the examined object **3,** while the projections under different angles are ensured by known methods, i.e. rotation of the examined object **3** placed on a turning table **29,** where the other elements, the source **1**, the grating **2** and the detector **7** are fixed, as seen in Fig. 7a, or by means of a system that contains the radiation source **1**, the grating **2** and the detector **7** located on a rotating gantry **30** rotating around the examined object **3** placed on a fixed table **31,** as seen in Fig. 7b.

The sum of signals generated by one particle which is captured by all the pixels **14** of the appropriate multiplet **13** determines the total energy of a particle **27** penetrated through the examined object **3** in the radiation microbeam. This fact can be used for energy-sensitive phase radiography if particles **27** of different energies are contained in individual microbeams.

### Industrial applicability

The radiographic system can be used for various radiation types such as X-ray radiation, gamma radiation, slow neutrons etc. The system can particularly be applied to X-ray phase sensitive radiography of living (in vivo and in vitro) and non-living samples. Typical field of application are mammography, defectoscopy, imaging of structures of alloys and composite materials, imaging of inner structure of archaeological samples, artefacts and cultural heritage objects. If the X-ray energy is controlled or directly measured, the invention is also applicable to energy-sensitive phase imaging enabling identification of materials contained in sample structure. The imaging system can be combined with computed tomography techniques (CT) and provide 3D imaging of internal structure of examined samples.

The invention can also be used for e.g. phase sensitive imaging by means of slow neutrons. Detector sensitivity to slow neutrons can be ensured by adding suitable conversion material such as ⁶Li or ¹⁰B, on the surface or inside the sensitive volume of the detector, which can be a semiconductor sensor or a scintillation screen. This method can be used for imaging metal structures, composite materials, mineral or geological samples, art objects and cultural heritage objects.

### LIST OF REFERENCE SIGNS

1. radiation source
2. directing element (grating)
3. examined object
4. detector grating
5. refracted beam (with the object)
6. non-refracted beam (without the object)
7. detector
8. difference in the position between the refracted and the non-refracted beam
9. characteristic intensity oscillation serving for phase shift evaluation observed in each pixel during the scanning of one of the gratings
10. phase gradient image
11. radiation from a spatially coherent source
12. width of the beam formed by the grating
13. pixel multiplet
14. pixel
15. direction of phase gradient detection sensitivity
16. the place of beam incidence on the detector
17. diameter of the track of the microbeam incident to the detector.
18. record of intensity (number of photons) of the incident radiation in individual pixels
19. record of the signal generated by each detected particle in individual pixels
20. common electrode
21. matrix of electrodes - pixels
22. semiconductor sensor
23. ionizing particle in the non-refracted beam
24. ionising particle penetration depth
25. ionising charge generated by interacting particle
26. read-out chip processing the size of signal generated by each detected particle (Timepix)
27. ionizing particle in the refracted beam
28. sensor with 3D electrodes
29. rotating table for placing the examined object
30. rotating gantry
31. fixed table for placing the examined object

### REFERENCES

[1] D. Christian, T. Weitkamp, F. Pfeiffer, Interferometer for quantitative phase contrast imaging and tomography with an incoherent polychromatic x-ray source, patent application EP 1 731 099 A1
[2] D. Christian, T. Donath,E. Hempel, M. Hoheisel, F. Pfeiffer, S. Popescu, X-ray CT system for X-ray phase contrast and/or X-ray dark field imaging, patent application US 2010/0091936 A1
[3] F. Pfeiffer, T. Weitkamp, O. Bunk, and C. David, Nat. Phys. 2, 258 (2006).
[4] T. Weitkamp, A. Diaz, C. David, F. Pfeiffer, M. Stampanoni, P. Cloetens and E. Ziegler, Opt. Express 13, 6296 (2005).
̌[5] F. Kreǰčí, J. Jakubek, M. Kroupa, " Hard x-ray phase contrast imaging using single absorption grating and hybrid semiconductor pixel detector", Rev. Sci. Instrum. 81, 113702 (2010).
[6] F. Talbot, "Facts relating to optical science no. IV," The London and Edinburgh Philosophical Magazine and Journal of Science, vol. 9, no. 56, pp. 401-407, 1836.
[7] E. Tkaczyk et al., "Method and apparatus to reduce charge sharing in pixellated energy discriminating detectos", patent application US7486764 B2 (2009).
[8] R. Ballabriga et al., "Medipix3: A 64 k pixel detector readout chip working in single photon counting mode with improved spectrometric performance", Nuc. Instr. and Meth. In Phys. Res. A, 633, Supplement 1, May 2011, Pages S15-S18
[9] J. Jakubek, A. Cejnarova, T. Holy, S. Pospisil, J. Uher, Z. Vykydal, "Pixel Detectors for Imaging with Heavy Charged Particles", Nucl. Instr. and Meth. A, Volume: 591, Issue:1, Pages: 155-158, doi: 10.1016/j.nima.2008.03.091 (2008).
[10] J. Jakubek, M. Platkevic, P. Schmidt-Wellenburg, P. Geltenbort, C. Plonka-Spehr, M. Daum, "Position-sensitive spectroscopy of ultra-cold neutrons with Timepix pixel detector", Nucl. Instr. and Meth. A, Vol. 607, Issue 1, p. 45-47 (2009).
[11] J. Jakubek, J. Dammer, T. Holy, M. Jakubek, S. Pospisil, V. Tichy, J. Uher, D. Vavrik, "Spectrometric Properties of TimePix Pixel Detector for X-ray Color and Phase Sensitive Radiography", IEEE NSS Conf. Proc. (2007) N50-6.
[12] X. Llopart, R. Ballabriga, M. Campbell, L. Tlustos, W. Wong, "Timepix, a 65k programmable pixel readout chip for arrival time, energy and/or photon counting measurements," Nuc. Instr. and Meth. In Phys. Res. A 581 (2007), 485-494.

## Claims

1. A radiation imaging method including the steps of generating at least one radiation microbeam by a radiation source, penetration of radiation through an examined object and its capture by a detector, wherein the difference between the position of a non-refracted microbeam detected without the examined object and the position of a refracted microbeam detected with the examined object defines a refraction angle in a particular point of the examined object, the refraction angle expressing the size of a local gradient of the refractive index corresponding to a phase gradient image (PGI) of the examined object, **characterized in that** the radiation on the way from the radiation source **(1)** to the detector **(7)** passes through a directing element **(2)** which transmits a radiation microbeam with crosswise dimension and directs the radiation microbeam to the boundaries between pixels **(14)** of the detector **(7),** wherein the incident radiation particle generates an analogue signal in the pixels **(14)** adjacent to the boundary and each of the pixels **(14)** detects the appropriate share of the signal, the signal share being defined as the effect of the same photo event causing a detected signal in a plurality of pixels **(14)** adjacent to the boundary, wherein the non-refracted microbeam generates approximately the same share of analogue signal in each pixel of a multiplet, and then after object insertion, the microbeam is refracted in the particular point of the object and the analogue signal is unequally distributed and shared among pixels adjacent to the boundary within one multiplet, and the difference **(8)** of the position between the refracted microbeam **(5)** and the non-refracted microbeam **(6)** is directly derived from the change of the share of analogue signal within the appropriate pixels **(14)** adjacent to the boundary, and thus the phase gradient in the particular object point is directly derived from the analogue signal.

2. The radiation imaging method according to claim 1, **characterized in that** the evaluation is performed for each individual detected particle in the refracted radiation microbeam **(5).**

3. The radiation imaging method for detecting spatial distribution of a refractive index in an examined object by means of a computed tomography method, wherein a set of projections is measured, namely the phase gradient images (PGIs) according to claim 1 or 2, obtained for various angles of rotation of the examined object (3), **characterized in that** the projections under various angles are ensured by rotation of the examined object **(3)** placed on a turning table **(29),** where the other elements being the source **(1),** the grating **(2)** and the detector **(7)** are fixed.

4. The radiation imaging method for detecting space distribution of a refractive index in an examined object by means of a computed tomography method, wherein a set of projections is measured, namely the phase gradient images (PGIs) according to claim 1 or 2, obtained for various angles of rotation of the examined object **(3), characterized in that** the projections under various angles are performed by the radiation source **(1),** the grating **(2)** and the detector **(7)** which are arranged on a rotating gantry **(30)** orbiting the examined object **(3)** located on a fixed table **(31).**

5. An arrangement of a radiographic imaging system for application of the method according to claims 1-4, consisting of the spatially coherent radiation source **(1),** space for positioning of the examined object **(3)** and the detector **(7)** consisting of the set of pixels **(14), characterized in that** it has the directing element **(2)** for directing the microbeams to the area of the boundary between at least two pixels **(14)** of the detector **(7)** to form a multiplet **(13),** wherein a crosswise dimension **(17)** of each microbeam of the radiation falling on the detector **(7)** is c times smaller than the size of the detector pixel **(14),** where c is 2 - 1000 and c > 20 is advantageous, and the size of pixels **(14)** is within the range of 0.1 - 500 µm, wherein the detector **(7)** is arranged to detect the share of signal defined as the effect of the same photo event causing a detected signal in a plurality of pixels **(14)** adjacent to the boundary.

6. The arrangement of the radiographic imaging system according to claim 5, **characterized in that** the directing element **(2)** is a grating or micro-capillary X-ray optics.

7. The arrangement of the radiographic imaging system according to claim 5 or 6, enabling identification of materials in the structure of the examined object **(3)** by evaluation of the applied radiation energy, **characterized in that** the radiation source **(1)** contains elements enabling to control energy of produced radiation or the detector **(7)** enables determination of the energy of each detected particle **(27).**

## Patentansprüche

1. Ein Strahlungsabbildungsverfahren umfassend die Schritte des Erzeugens mindestens eines Strahlungsmikrostrahls durch eine Strahlungsquelle, des Eindringens von Strahlung durch ein untersuchten Objekt und ihrer Aufnahme durch einen Detektor, wobei die Differenz zwischen der Position eines ungebrochenen Mikrostrahls, der ohne das untersuchten Objekt detektiert wird, und der Position eines gebrochenen Mikrostrahls, der mit dem untersuchten Objekt detektiert wird, einen Brechungswinkel in einem bestimmten Punkt des untersuchten Objekts definiert, wobei der Brechungswinkel die Größe eines lokalen Gradienten des Brechungsindex äußert, der einem Phasengradientenbild (PGB) des untersuchten Objekts entspricht, **dadurch gekennzeichnet, dass** die Strahlung auf dem Weg von der Strahlungsquelle (1) zu dem Detektor (7) durch ein Leitelement (2) hindurchtritt, der einen Strahlungsmikrostrahl mit Querabmessung aussendet und den Strahlungsmikrostrahl auf die Grenzen zwischen Pixeln (14) des Detektors (7) lenkt, wobei das einfallende Strahlungspartikel ein analoges Signal in den an der Grenze benachbarten Pixeln (14) erzeugt und jedes Pixel (14) den entsprechenden Signalanteil erfasst, wobei der Signalanteil als der Effekt des gleichen Fotoereignisses definiert ist, das ein erfasstes Signal in einer Vielzahl von an der Grenze benachbarten Pixeln (14) verursacht, wobei der ungebrochene Mikrostrahl in jedem Pixel eines Multiplets annähernd den gleichen Anteil des analogen Signals erzeugt, und dann nach dem Einsetzen des Objekts, der Mikrostrahl in dem jeweiligen Punkt des Objekts gebrochen wird und das analoge Signal ungleichmäßig verteilt und zwischen Pixeln neben der Grenze innerhalb eines Multiplets geteilt wird und die Differenz (8) der Position zwischen dem gebrochenen Mikrostrahl (5) und dem ungebrochenen Mikrostrahl (6) direkt aus der Änderung des Anteils des analogen Signals innerhalb der entsprechenden, an der Grenze benachbarten Pixel (14) abgeleitet wird und somit der Phasengradient in dem jeweiligen Punkt des Objektes direkt aus dem analogen Signal abgeleitet wird.

2. Das Strahlungsabbildungsverfahren nach Anspruch 1, **dadurch gekennzeichnet, dass** die Auswertung für jedes einzelne detektierte Partikel im gebrochenen Strahlungsmikrostrahl (5) durchgeführt wird.

3. Das Strahlungsabbildungsverfahren zur Erfassung der räumlichen Verteilung eines Brechungsindex in einem untersuchten Objekt mittels eines Computertomographieverfahrens, wobei ein Satz von Projektionen gemessen wird, nämlich die Phasengradientenbilder (PGB) nach Anspruch 1 oder 2, die für verschiedene Drehwinkel des untersuchten Objekts (3) erhalten sind, **dadurch gekennzeichnet, dass** die Projektionen unter verschiedenen Winkeln durch Drehung des auf einem Drehtisch (29) angeordneten untersuchten Objekts (3) sichergestellt werden, wobei die anderen Elemente - die Quelle (1), das Gitter (2) und der Detektor (7) fixiert sind.

4. Das Strahlungsabbildungsverfahren zur Erfassung der räumlichen Verteilung eines Brechungsindex in einem untersuchten Objekt mittels eines Computertomographieverfahrens, wobei ein Satz von Projektionen gemessen wird, nämlich die Phasengradientenbilder (PGB) nach Anspruch 1 oder 2, die für verschiedene Drehwinkel des untersuchten Objekts (3) erhalten sind, **dadurch gekennzeichnet, dass** die Projektionen unter verschiedenen Winkeln von der Strahlungsquelle (1), dem Gitter (2) und dem Detektor (7) durchgeführt werden, die auf einem rotierenden Portal (30) angeordnet sind, das das auf einem feststehenden Tisch (31) befindliche, untersuchte Objekt (3) umkreist.

5. Eine Anordnung eines Radiografie-Abbildungssystems zur Anwendung des Verfahrens nach den Ansprüchen 1-4, bestehend aus der räumlich kohärenten Strahlungsquelle (1), einem Raum für die Positionierung des untersuchten Objektes (3) und dem Detektor (7) bestehend aus dem Satz von Pixeln (14), **dadurch gekennzeichnet, dass** sie das Leitelement (2) zum Richten der Mikrostrahlen auf den Bereich der Grenze zwischen mindestens zwei Pixeln (14) des Detektors (7) zu einem Multiplet (13) aufweist, wobei eine Querabmessung (17) jedes Mikrostrahls der auf den Detektor (7) fallenden Strahlung c-mal kleiner ist als die Größe des Pixels (14) des Detektors, wobei c 2-1000 ist und vorzugsweise c > 20 ist, und die Größe der Pixel (14) im Bereich von 0,1-500 µm liegt, wobei der Detektor (7) eingerichtet ist, den Signalanteil zu erfassen, der als der Effekt des gleichen Fotoereignisses definiert ist, das ein erfasstes Signal in einer Vielzahl von an der Grenze benachbarten Pixeln (14) verursacht.

6. Die Anordnung des Radiografie-Abbildungssystems nach Anspruch 5, **dadurch gekennzeichnet, dass** das Leitelement (2) ein Gitter oder eine Mikrokapillar-Röntgenoptik ist.

7. Die Anordnung des Radiografie-Abbildungssystems nach Anspruch 5 oder 6, die eine Identifizierung von Materialien in der Struktur des untersuchten Objekts (3) durch Auswertung der angewandten Strahlungsenergie ermöglicht, **dadurch gekennzeichnet, dass** die Strahlungsquelle (1) Elemente enthält, die es ermöglichen, Energie erzeugter Strahlung zu steuern, oder der Detektor (7) eine Bestimmung der Energie jedes detektierten Partikels (27) ermöglicht.

## Revendications

1. Un procédé d'imagerie par rayonnement comprenant les étapes de génération au moins un microfaisceau de rayonnement par une source de rayonnement, de pénétration de rayonnement à travers un objet examiné et de capture de celui-ci par un détecteur, où la différence entre la position d'un microfaisceau non-réfracté détecté sans l'objet examiné et la position d'un microfaisceau réfracté détecté avec l'objet examiné définit un angle de réfraction dans un point particulier de l'objet examiné, où l'angle de réfraction exprime la taille d'un gradient local d'un indice de réfraction correspondant à une image à gradient de phase (IGP) de l'objet examiné, **caractérisé en ce que** le rayonnement sur la voie de la source de rayonnement (1) au niveau du détecteur (7) passe à travers un élément d'orientation (2) qui transmet un microfaisceau de rayonnement avec une dimension transversale et dirige le microfaisceau de rayonnement vers les frontières entre les pixels (14) du détecteur (7), où la particule de rayonnement incident génère un signal analogique dans les pixels (14) adjacents à la frontière et à chacun des pixels (14) détecte la part appropriée du signal, où la part du signal est définie comme l'effet du même photo-événement provoquant un signal détecté dans une pluralité de pixels (14) adjacents à la frontière, où le microfaisceau non-réfracté génère approximativement la même part de signal analogique dans chaque pixel d'un multiplet, et puis après l'insertion de l'objet, le microfaisceau est réfracté dans le point particulier de l'objet et le signal analogique est distribué et partagé de manière irrégulière parmi les pixels adjacents à la frontière dans un multiplet, et la différence (8) de la position entre le microfaisceau réfracté (5) et le microfaisceau non-réfracté (6) est directement dérivée du changement de la part du signal analogique dans les pixels appropriés (14) adjacents à la frontière, et ainsi le gradient de phase dans le point d'objet particulier est directement dérivé du signal analogique.

2. Le procédé d'imagerie par rayonnement selon la revendication 1, **caractérisé en ce que** l'évaluation est effectuée pour chaque particule individuelle détectée dans le microfaisceau de rayonnement réfracté (5).

3. Le procédé d'imagerie par rayonnement pour détecter la distribution spatiale d'un indice de réfraction dans un objet examiné au moyen d'un procédé de tomographie assistée par ordinateur, où un ensemble de projections est mesuré, à savoir les images à gradient de phase (IGP) selon la revendication 1 ou 2, obtenues pour divers angles de rotation de l'objet examiné (3), **caractérisé en ce que** les projections sous divers angles sont assurées par la rotation de l'objet examiné (3) placé sur une table tournante (29), où les autres éléments étant la source (1), le réseau (2) et le détecteur (7) sont fixés.

4. Le procédé d'imagerie par rayonnement pour détecter la distribution spatiale d'un indice de réfraction dans un objet examiné au moyen d'un procédé de tomographie assistée par ordinateur, où un ensemble de projections est mesuré, à savoir les images à gradient de phase (IGP) selon la revendication 1 ou 2, obtenues pour divers angles de rotation de l'objet examiné (3), **caractérisé en ce que** les projections sous divers angles sont réalisées par la source de rayonnement (1), le réseau (2) et le détecteur (7) disposés sur un portique rotatif (30) en orbite autour de l'objet examiné (3) situé sur une table fixe (31).

5. Un agencement d'un système d'imagerie radiographique pour l'application du procédé selon les revendications 1 à 4, constitué de la source de rayonnement (1) spatialement cohérente, de l'espace pour placer l'objet examiné (3) et du détecteur (7) constitué de l'ensemble de pixels (14), **caractérisé en ce qu'**il comporte l'élément d'orientation (2) pour diriger les microfaisceaux vers la zone de la frontière entre au moins deux pixels (14) du détecteur (7) pour former un multiplet (13), où une dimension transversale (17) de chaque microfaisceau du rayonnement tombant sur le détecteur (7) est c fois inférieure à la taille du pixel de détecteur (14), c étant de 2 à 1000 et c > 20 étant avantageux, et la taille des pixels (14) est dans la gamme de 0,1 à 500 µm, où le détecteur (7) est disposé pour détecter la part du signal défini comme l'effet du même photo-événement provoquant un signal détecté dans une pluralité de pixels (14) adjacents à la frontière.

6. L'agencement du système d'imagerie radiographique selon la revendication 5, **caractérisé en ce que** l'élément d'orientation (2) est un réseau ou une optique à rayons X à micro-capillaire.

7. L'agencement du système d'imagerie radiographique selon la revendication 5 ou 6, permettant l'identification de matériaux dans la structure de l'objet examiné (3) par évaluation de l'énergie de rayonnement appliquée, **caractérisé en ce que** la source de rayonnement (1) contient des éléments permettant de commander l'énergie du rayonnement produit ou le détecteur (7) permet de déterminer l'énergie de chaque particule détectée (27).
